# EUROPÄISCHE PATENTSCHRIFT

(11) **EP 0 497 915 B1**
(45) Veröffentlichungstag und Bekanntmachung des Hinweises auf die Patenterteilung: **19.04.1995**
(21) Anmeldenummer: 90917747.9
(22) Anmeldetag: 25.10.1990
(51) Int. Cl.: C07K 14/10, A61K 38/22, G01N 33/74

(54) **hPTH-FRAGMENT-(1-37), SEINE HERSTELLUNG, DIESES ENTHALTENDE ARZNEIMITTEL UND SEINE VERWENDUNG**
hPTH (1-37) FRAGMENT, ITS PRODUCTION, DRUG CONTAINING IT AND ITS USE
FRAGMENT DE hPTH (1-37), SA PRODUCTION, MEDICAMENT LE CONTENANT ET SON UTILISATION

(30) Priorität: 27.10.1989 DE 3935738
(43) Veröffentlichungstag der Anmeldung: 12.08.1992
(73) Patentinhaber: HaemoPep Pharma GmbH, D-30625 Hannover (DE)
(72) Erfinder: FORSSMANN, Wolf-Georg, D-3000 Hannover 1 (DE); HERBST, Franz, D-6907 Nussloch (DE); SCHULZ-KNAPPE, Peter, D-6730 Neustadt a.d.W. (DE); ADERMANN, Knut, D-3000 Hannover 61 (DE); GAGELMANN, Michael, D-6905 Schriersheim (DE)
(74) Vertreter: Werner, Hans-Karsten, Dr.Dipl.-Chem.
(86) Internationale Anmeldenummer: EP9001807
(87) Internationale Veröffentlichungsnummer: WO9106564

(56) Entgegenhaltungen:
- EP-A- 0 110 294
- EP-A- 0 293 159
- CHEMICAL ABSTRACTS, vol. 102, no. 17, 29 April 1985, Columbus, OH (US); J.E. ZULL et al., p. 97, no. 143622q#
- CHEMICAL ABSTRACTS, vol. 106, no. 5, 02 February 1987, Columbus, OH (US); S. PILLAY et al., p. 92, no. 280085n#
- PATENT ABSTRACTS OF JAPAN, vol. 7, no. 39 (C151), 26 November 1982; Tanpakushitsu Kenkiyuu Shiyoureikai#

## Beschreibung

Die vorliegende Erfindung betrifft ein hPTH-Fragment-(1-37), seine Herstellung, dieses enthaltende Arzneimittel und seine Verwendung.

Humanes Parathormon (hPTH), das Hormon der Nebenschilddrüsen, stellt ein potentiell wichtiges therapeutisches Hilfsmittel, z.B. für die Behandlung der Osteoporose und des Hypoparathyreoidismus, dar.

Die Osteoporose (Verminderung der Knochenmasse) (*Riggs und Melton, N. Engl. J. Med*. *314 1676-1686, 1986)* ist eine häufige Krankheit, von der vor allem Frauen in der Menopause und ältere Menschen betroffen sind. Die Betroffenen leiden je nach Ausprägung unter häufigen Frakturen im Bereich der Wirbelsäule, der Unterarme oder Oberschenkel, Schmerzen bis zur völligen Unbeweglichkeit, Verlust der Arbeitsfähigkeit und sozialer Kontakte sowie einem höheren Sterblichkeitsrisiko. Eine Heilung gilt derzeit als kaum möglich (*Consensus Development Conference: Prophylaxis and Treatment of Osteoporosis*, *1987*). Tierexperimentelle Untersuchungen (*Selye, Endocrinolog*y, *16, 547-558, 1932*; *Kalu et al., Lancet 1363-1366, 1970*; *Hefti et al*., *Clin. Science 62, 389-396, 1982*; *Tam et al*., *Endocrinology 110, 506-512, 1982*; *Podbesek et al*., *Endocrinology 112, 1000*-*1006, 1983*; *Gunness-Hey und Hock, Metab. Bone Dis. & Rel. Res. 5, 177-181*, *1984*) sowie neuere histomorphometrische Befunde beim primären Hyperparathyreoidismus (*Delling et al*., *Klin. Wochenschr. 65, 643-653, 1987*) legen die Vermutung nahe, daß durch eine Behandlung mit PTH eine Zunahme der Knochenmasse erzielt werden kann. Reeve et al. (*Br. Med. J*. *1340-134, 1980*) erreichten bei Patienten mit Osteoporose durch täglich verabreichte kleine Dosen von PTH tatsächlich eine Verbesserung der trabekulären Knochenstruktur bei jedoch leicht abnehmender corticaler Knochenmasse. Neuere Befunde sprechen dafür, daß PTH bei zusätzlicher Verabreichung von knochenaktiven Substanzen, wie z.B. 1,25-Vitamin-D₃ (*Slovik et al., J. Bone Miner*. *Res. 1, 377-381, 1986*), Calcitonin (*Hesch et al., Calcif. Tissue Int. 44, 176-180, 1989*) oder Östrogen (*Reeve et al*., *Proceedings of the 5th International Congress on Bone Morphometry, Niigata, 24.*-*29.7. 1988*), die Knochenmasse der Spongiosa ohne Corticalis-Verlust erhöht.

Der Hypoparathyreoidismus (PTH-Mangel) (*Kruse, Monatsschr. Kinderheilkunde 136, 652-666, 1988*) tritt entweder congenital oder als Folge einer Operation oder von Bestrahlungen im Halsbereich auf und führt zu einer erniedrigten Calcium-Konzentration im Blut. Die Patienten neigen zu Krampfanfällen. Besteht der PTH-Mangel bereits im Kindesalter, drohen langfristig verminderte geistige Entwicklung und eine defekte Zahn- und Knochenentwicklung. Eine Therapie mit Calcium- und/oder Vitamin-D-Präparaten normalisiert zwar bei den meisten Patienten die Calcium-Konzentration im Serum, ist jedoch mit einem erhöhten Risiko von Nierenschädigungen verbunden. Dieses Risiko einer medikamentösen Behandlung kann durch eine Hormonsubstitutionstherapie mit PTH vermieden werden.

Schließlich hat sich in jüngster Zeit gezeigt, daß das Parathyreoidhormon blutdrucksenkende Wirksamkeit aufweist (*Nickols, Blood Vessels 24, 120-124, 1987*).

Sowohl bei der Behandlung von Osteoporose und Hypertonus als auch bei der Hormonsubstitution bei Hypoparathyreoidismus muß das PTH regelmäßig über einen langen Zeitraum, gegebenenfalls lebenslang, verabreicht werden. Das verwendete PTH muß deshalb frei von Verunreinigungen sein und darf nicht die Bildung von Antikörpern hervorrufen. Diesen Anforderungen kann am wirksamsten durch chemisch oder gentechnologisch synthetisierte Peptide mit der Aminosäuresequenz des menschlichen PTH entsprochen werden. Das sezernierte PTH-Molekül besteht aus 84 Aminosäuren [hPTH-(1-84)] dieser Größenordnung sind einer chemischen Synthese jedoch nur schwer zugänglich und besser gentechnologisch herstellbar.

Bisher sind zwei verschiedene Peptide mit den aminoterminalen Teilsequenzen des humanen PTH - das hPTH-(1-34) und das hPTH-(1-38) - synthetisiert worden. Bei der klinischen Erprobung im Rahmen einer Einmalinjektion für eine diagnostische Anwendung zeigten sich sowohl bei hPTH-(1-34) (*Mallette et al., J. Clin Endocrin.* Metab*. 67, 964-972, 1988*) als auch bei hPTH-(1-38) (*Kruse und Kracht, Eur. J. Pediatr. 146, 373-377, 1987*) die aufgrund früherer Erfahrungen mit extrahiertem Rinder-PTH (bPTH) erwarteten kurzfristigen Effekte, d.h. vorübergehende Stimulation der Ausscheidung von Phosphat und cyclischem Adenosinmonophosphat (cAMP) im Urin und vorübergehende Erhöhung der cAMP-Konzentration im Plasma.

Bei der therapeutischen Erprobung dieser Peptide bei einer kleinen Anzahl von Osteroporose-Patienten konnten sowohl mit hPTH-(1-34) (*Reeve et al., Proceedings of the 5th International Congress on Bone Morphometry, Niigata, 24.-29.7.1988*) als auch mit hPTH-(1-38) (*Hesch et al., Calcif. Tissue Int 44, 176-180, 1989*) gewisse Erfolge erzielt werden.

**Nachteilig an den bisher** für die klinische Anwendung verfügbaren PTH-Fragmenten ist jedoch, daß sie in einigen Patienten die **Bildung von Antikörpern hervorrufen**, welche die **Wirkung** der exogen zugeführten Fragmente oder sogar des körpereigenen PTH **aufheben** können [vgl. beispielsweise für hPTH-(1-34), *Audran et al., J. Clin. Endocrinol. Metab. 64, 937-943, 1987* und für hPTH-(1-38) *Stögmann et al., Monatsschr. Kittderheilk. 136, 107 1988*].

Vor etwa 20 Jahren wiesen Berson und Yalow (*j. Clin Endocrinol. Metab. 28*, *1037-1047, 1968*) nach, daß verschiedene PTH-Fragmente im menschlichen Plasma vorkommen, die entweder durch raschen peripheren Abbau des Gesamtmoleküls oder aber durch Sekretion von PTH-Fragmenten entstanden sein konnten. Es zeigte sich, daß die in der Zirkulation mengenmäßig überwiegenden Produkte des peripheren PTH-Metabolismus große carboxylterminale PTH-Fragmente ohne biologische Aktivität sind, die in der Leber gebildet werden (beispielsweise *D'Amour und Huet, Am. J. Physiol 246, E249-255, 1984*). Aufgrund der vorliegenden Untersuchungsergebnisse wurde angenommen, daß bei intakter Nierenfunktion das hPTH-(1-84) die dominierende biologisch aktive PTH-Form ist. Bei eingeschränkter Nierenfunktion trat hingegen in fraktioniertem Plasma der jeweiligen Patienten in der Elutionsposition des hPTH-(1-34) ein deutlicher Gipfel in Erscheinung (vgl. *Grunbaum et al., Am. J. Physiol*. *247, E442-448, 1984*).

**Die Existenz eines biologisch aktiven, im menschlichen Plasma gesunder Patienten zirkulierenden PTH-Fragments konnte bisher nicht nachgewiesen werden.**

Es ist daher verschiedentlich versucht worden, den endogenen Abbau des PTH durch *in vitro* Untersuchungen zu simulieren, um auf diese Weise Rückschlüsse auf primäre Spaltstellen in dem Gesamtmolekül zu ziehen. In diesem Zusammenhang wurde nahezu jede Position zwischen den Aminosäuren Nr. 5 und Nr. 43 des aminoterminalen Endes in Betracht gezogen (vgl. beispielsweise *Barling et al., Int. J. Biochem*., *16, 815-821, 1984*). Unter anderem führten *Zull und Chuang (J. Biol. Chem*. *260, 1608-1613, 1985*) Untersuchungen mit bPTH und Cathepsin D durch. Sie konnten nachweisen, daß bei der enzymatischen Spaltung von Rinder-PTH (bPTH) mit dem aus Rindermilz stammenden Enzym die C-terminalen Fragmente (35-84) und (38-84) sowie die komplementären N-terminalen Fragmente (1-34) und (1-37) gebildet werden. Gegenüber dem von uns gefundenen Fragment hPTH-(1-37) unterscheidet sich das in dieser Arbeit beschriebene bPTH in den Positionen 1 (Ser anstatt Ala), 7 (Leu anstatt Phe) und 16 (Asn anstatt Ser). Die letztgenannten, bovinen Fragmente erwiesen sich bei *in vitro* Untersuchungen mit Nierenmembranen aus Ratte und Rind als biologisch aktiv, wobei jedoch das Fragment-(1-37) nur in sehr geringen Mengen nachweisbar war und schnell zu Fragment-(1-34) hydrolysiert wurde. Die Autoren schlossen daraus, daß das Endprodukt des enzymatischen Abbaus von PTH durch Cathepsin D beim Rind das Fragment-(1-34) ist. Demgegenüber bezweifelten andere Autoren, daß in vivo überhaupt N-terminale PTH-Fragment im Plasma erscheinen (vgl. beispielsweise *Goltzman et al., J. Clin. Invest. 65, 1309-1317, 1980*). So konnten bei normalen Ratten keine durch peripheren Abbau gebildeten, biologisch aktiven, N-terminalen PTH-Fragmente nachgewiesen werden (*Bringhurst et al., Am. J. Physiol. 255, E886-893, 1982*). *MacGregor et al*., (*J. Biol. Chem*. *261, 1929-1934, 1986*) fanden, daß bovine Nebenschilddrüsen in Kultur keine biologisch aktiven, N-terminalen PTH-Fragmente sezernieren. **Zusammengefaßt legten diese Befunde zum Zeitpunkt der vorliegenden Erfindung nahe, daß im menschlichen oder tierischen Blutplasma keine biologisch aktiven, N-terminalen PTH-Fragmente zirkulieren.**

Es ist **Aufgabe der vorliegenden Erfindung**, ein neues hPTH-Fragment-(1-37) bereitzustellen, das ein gut zugängliches Arzneimittel mit der biologischen und therapeutischen Aktivität von natürlichem Parathormon (PTH) ist, welches mindestens den Wirkungsgrad der bekannten hPTH-Fragmente (1-34) und (1-38) aufweist und deren Nachteile - insbesondere die Induktion der Antikörperbildung - vermeidet.

Weitere Aufgabe der vorliegenden Erfindung ist die Bereitstellung eines Herstellungsverfahrens für dieses hPTH-Fragment-(1-37) sowie die Anwendung als Arzneimittel für verschiedene therapeutische und diagnostische Indikationen.

Diese Aufgaben werden durch ein hPTH-Fragment mit neuer Aminosäurensequenz gelöst.

### Die vorliegende Erfindung betrifft somit:

Ein hPTH-Fragment-(1-37) mit der Aminosäurensequenz 
Ser - Val - Ser - Glu - Ile
- Gln - Leu - Met - His - Asn - Leu - Gly - Lys - His - Leu - Asn - Ser - Met - Glu -
Arg - Val - Glu - Trp - Leu - Arg - Lys - Lys - Leu - Gln - Asp - Val - His - Asn -
Phe - Val - Ala - Leu
sowie seine natürlichen und pharmakologisch verträglichen Derivate, insbesondere amidierte, acetylierte, phosphorylierte und glycosylierte hPTH-(1-37)-Derivate.

Die vorliegende Erfindung betrifft weiter ein Verfahren zur Herstellung dieses hPTH-Fragmentes-(1-37) oder seiner Derivate dadurch gekennzeichnet, daß dieses über eine prokaryontische oder eine eukaryontische Expression hergestellt und chromatographisch gereinigt wird, sowie ein weiteres Verfahren zur Herstellung des hPTH-Fragmentes-(1-37) oder seiner Derivate, indem man es aus menschlichem Blut über Chromatographie-Verfahren in bekannter Weise isoliert, und schließlich ein Verfahren zur Herstellung des hPTH-Fragmentes-(1-37) oder seiner Derivate, indem man dieses hPTH-Fragment durch die üblichen Verfahren der Festphasen- und Flüssigphasen-Synthese aus den geschützten Aminosäuren, die in der angegebenen Sequenz enthalten sind, herstellt, deblockiert und es mit den gängigen Chromatographie-Verfahren reinigt.

Erfindungsgemäß hat es sich überraschenderweise gezeigt, daß das kürzeste hPTH-Fragment mit voller biologischer Aktivität, welches durch primäre Spaltung des hPTH-(1-84) im menschlichen Körper entsteht, das hPTH-(1-37) ist (vgl. Beispiel 1). Überraschenderweise zeigte sich auch, daß sich die Raumstruktur von hPTH-(1-37) deutlich von den bisher bekannten Fragmenten unterscheidet (vgl. Beispiel 5), was auf seine besonderen Struktur-Wirkungs-Beziehungen und Antigen-Eigenschaften hinweist.

Das hPTH-Fragment-(1-37) wurde chemisch synthetisiert (vgl. Beispiel 2) und als Arzneimittel zubereitet. Auch die gentechnologische Herstellung über übliche Vektoren ist erarbeitet: auf gentechnologischem Wege wird das hPTH-(1-37) Peptid sowohl (1) in pro- als auch (2) in eukaryontischen Organismen hergestellt. Für die prokaryontische Expression benutzen wir vorzugsweise Escherichia coli. Hierfür stehen u.a. Expressionsvektoren zur sekretorischen Expression (z.B. pSP6, pRit-Derivate, Pharmacia), zur direkten cytoplasmatischen Expression (z.B. pKK-Derivate, Pharmacia) oder Expression als Fusionsprotein (pMC 1871, Pharmacia) zur Verfügung (Literatur siehe bei *Marston et al., Biochem. J.*, *240, 1-12, 1986*). Für die eukaryontische Expression haben wir verschiedene Organismen und Vektoren zur Verfügung, z.B. Insektenzellen (*Summers and Smith, Tex.Agric.Exp.Stn.(bull) 1555, 1987*), Hefen (*Hitzeman et al., Nature*, *293, 717-722, 1981)*, filamentöse Pilze (*Yelton et al., Proc.Natl.Acad.Sci.USA*, *81, 1470-1474, 1984)*) und Säugerzellen (*Zettlmeissl et al*., *Biotechnology*, *5, 720-725*, *1987)*, von denen wir vorzugsweise die Insektenzellen benutzen. Das exprimierte Peptid wird mit Methoden der Chromatographie gereinigt, vorzugsweise wie in Beispiel 1 angegeben.

Die Arzneimittelzubereitung enthält hPTH-(1-37) oder ein physiologisch verträgliches Salz des hPTH-(1-37). Die Form und Zusammensetzung des Arzneimittels, welches das hPTH-(1-37) enthält, richtet sich nach der Art der Verabreichung. Das humane PTH-(1-37) kann parenteral, intranasal, oral und mittels Inhalation verabreicht werden. Vorzugsweise wird hPTH-(1-37) zu einem Injektionspräparat, entweder als Lösung oder als Lyophilisat zur Auflösung unmittelbar vor Gebrauch, konfektioniert. Die Arzneimittelzubereitung kann außerdem Hilfsstoffe enthalten, die abfülltechnisch bedingt sind, einen Beitrag zur Löslichkeit, Stabilität oder Sterilität des Arzneimittels leisten oder den Wirkungsgrad der Aufnahme in den Körper erhöhen. Die zu verabreichende Tagesdosis hängt von der Indikation ab. Bei der Therapie der Osteoporose durch i.v./i.m. Injektion liegt sie im Bereich von 100 bis 1200 Einheiten (»g)/Tag, bei täglicher subcutaner Injektion vorzugsweise bei 300 - 2400 Einheiten (»g)/Tag. Die Bestimmung der biologischen Aktivität basiert auf Messungen gegen internationale und hauseigene Referenzpräparationen für humane PTH-Fragmente in einem gebräuchlichen biologischen Testverfahren für hPTH-Fragmente.

Das erfindungsgemäße Fragment hPTH-(1-37) eignet sich in besonderer Weise als Langzeit-Therapeutikum bei Hypoparathyreoidismus und Osteoporose, da es über eine ausgezeichnete biologische Wirksamkeit verfügt und andererseits auch bei lebenslanger Dauerbehandlung keine Immunreaktion auslöst.

Das erfindungsgemäße Präparat ist ferner als Blutdruck-stabilisierendes Mittel und insbesondere zur Langzeit- bzw. Dauerbehandlung bei essentieller Hypertonie geeignet.

Das erfindungsgemäße Präparat ist weiter als Mittel zur Therapie von Nierenerkrankungen, in der Intensivpflege und bei Lungenerkrankungen anzuwenden (vgl. Beispiel 6).

Das erfindungsgemäße Präparat ist weiter als Mittel zur Therapie der männlichen Impotenz einzusetzen (vgl. Beispiel 6).

### Die Erfindung wird nachfolgend anhand von Beispielen und den folgenden Abbildungen, auf die in den Beispielen Bezug genommen wird, erläutert:

Es zeigen:
Abb. 1: Sephadex® G 25 - Präparative Large-Scale-Gel-Chromatographie des Alginsäure-Eluats zur Grobauftrennung nach Molekulargewicht und zur Entsalzung des Rohpeptidextraktes. Im schraffierten Bereich befinden sich die durch RIA bestimmten hPTH-Fragmente.
   - Säule:: Pharmacia K 100/100; ID 10 cm x 80 cm
   - Material:: Sephadex® G 25 medium
   - Eluent:: 1 M Essigsäure
   - Flußrate:: 5 ml/min
   - Absorption:: 280 nm
Abb. 2: Präparative HPLC-Kationen-Austauscher-Chromatographie zur weiteren Auftrennung des Peptidmaterials aus Abbildung 1. Fraktionen 2 bis 5 von jeweils 16 ml enthalten je Fraktion mehr als 640 pMol hPTH-(44-68)-IR Material (schraffiert).
   - Säule:: HPLC-Stahlsäule 2 cm x 10 cm
   - Material:: Parcosil Pepkat
   - Eluent:: A: 5 mM K_{*2*}HPO_{*4*} pH 3,0; B: wie A in 1 M NaCl
   - Flußrate:: 8 ml/min
   - Absorption:: 280 nm
   - Gradient:: 0 - 60 % B in 60 min
Abb. 3: Semipräparative RP-Chromatographie zur Auftrennung der Fraktionen 2 bis 5 aus Abbildung 2. In Fraktion 17 und Fraktion 20 von je 3 ml befinden sich jeweils mehr als 180 pMol durch RIA auf hPTH-(44-68) nachweisbare hPTH-Fragmente:
   - Säule:: HPLC-Stahlsäule 1 cm x 10 cm
   - Material:: Orpegen RP HD-Gel 7/300
   - Eluent:: A: 0,01 M HCl; B: wie A in 80 % Acetonitril
   - Flußrate:: 3 ml/min
   - Absorption:: 280 nm
   - Gradient:: 0 - 60% B in 60 min
   - Temperatur:: 45° C
Abb. 4: Aufarbeitung der Fraktion 20 aus Abbildung 3 mit semipräparativer Kationen-Austauscher-Chromatographie. Fraktionen 32 und 33 von je 10 ml enthalten 750 bzw. 600 pMol eines hPTH-Fragmentes, das mit dem RIA auf hPTH-(44-68) nachweisbar ist.
   - Säule:: HPLC-Stahlsäule 1 cm x 5 cm
   - Material:: Parcosil Pepkat
   - Eluent:: A: 5 mM K_{*2*}HPO_{*4*} pH 3,0; B: wie A in 1 M NaCl
   - Flußrate:: 3 ml/min
   - Absorption:: 230 nm
   - Gradient:: 0 - 50 % B in 50 min.
Abb. 5: Zwischenschritt 1: Analytische RP-Chromatographie der Fraktionen 32 und 33 aus Abbildung 4. Es sind zwei Molekülformen von hPTH-(44-68)-IR nachweisbar, nämlich in Fraktion 12 von 3 ml mit über 150 pMol und in Fraktion 15 und 16 von je 3 ml mit jeweils über 300 pMol.
   - Säule:: HPLC-Stahlsäule 1 cm x 10 cm
   - Material:: Orpegen RP HD-Gel 7/300
   - Eluent:: A: 0,1 % TFA; B: wie A in 80 % Acetonitril
   - Flußrate:: 3 ml/min
   - Absorption:: 280 nm
   - Gradient:: 0 - 40 % B in 60 min
   - Temperatur:: 45° C
Abb. 6: Zwischenschritt 2: Analytische Kationen-Austauscher-Chromatographie der Frakionen 15 und 16 aus Abbildung 5. Die hPTH-(44-68)-IR Substanz ist besonders in Fraktion 9 von 2 ml (über 200 pMol) konzentriert.
   - Säule:: HPLC-Stahlsäule 0,5 cm x 5 cm
   - Material:: Parcosil Pepkat
   - Eluent:: A: 5 mM K_{*2*}HPO_{*4*} ph 3,0; B: wie A in 1 M NaCl
   - Flüßrate:: 0,7 ml/min
   - Absorption:: 230 nm
   - Gradient:: 0 - 50 % B in 50 min
Abb. 7: Hydrophobe-Interaktions-Chromatographie der Fraktionen 8 und 9 aus Abbildung 6. Die Fraktionen 5 bis 10 von je 0,87 ml enthalten das hPTH-(44-68)-IR Material in Konzentrationen von jeweils über 170 pMol/Fraktion.
   - Säule:: HPLC-Stahlsäule 0,5 cm x 5 cm
   - Material:: Parcosil Pro HIC
   - Eluent:: A: 100 mM Na_{*2*}HPO_{*4*} pH 6,5 B: wie A in 3 M (NH_{*4*})_{*2*}SO_{*4*}
   - Flußrate:: 0,7 ml/min
   - Absorption:: 230 nm
   - Gradient:: 100-0 % B in 45 min
Abb. 8: Analytische RP-Chromatographie der Fraktionen 5 bis 10 aus Abbildung 7. Es zeigt sich ein Hauptpeak mit der hPTH-(44-68)-IR.
   - Säule:: HPLC-Stahlsäule 0,5 x 10 cm
   - Material:: Orpegen RP HD-Gel 7/300
   - Eluent:: A: 0,1 % TFA; B: wie A in 80 % Acetonitril
   - Flüßrate:: 0,7 ml/min
   - Absorption:: 230 nm
   - Gradient:: 0 - 40 % B in 60 min
   - Temperatur:: 25° C
Abb. 9: Analytische RP-Chromatographie zur Endreinigung des hPTH-Fragmentes. Das Material der Fraktion 25 aus Abbildung 8 wurde rechromatographiert, und es ergibt sich im Hauptpeak ein hochreines Peptid, dessen Sequenz als hPTH-(38-84) bestimmt wurde.
   - Säule:: HPLC-Stahlsäule 0,5 cm x 10 cm
   - Material:: Orpegen RP HD-Gel 7/300
   - Eluent:: A: 0,1 % TFA; B: wie A in 80 % Acetonitril
   - Flüßrate:: 0,7 ml/min
   - Absorption:: 230 nm
   - Gradient:: 0 - 40 % B in 60 min
   - Temperatur:: 45° C
Abb. 10: Reversed-Phase-HPLC-Chromatographie der synthetischen hPTH-Fragmente aus dem N-terminalen Bereich, nämlich hPTH-(1-33), hPTH-(1-34), hPTH-(1-37), hPTH-(1-38). Werden diese Fragmente als Referenz für natürliche, im Blut zirkulierende Fragmente des N-Terminus von hPTH benutzt, läßt sich darstellen, daß das hPTH-(1-37) die korrekte Molekülform im humanen Blut ist.
   - Säule:: Parcosil ProRP 300 - 7, C4, 125 x 4 mm
   - Temperatur:: 55° C
   - Eluent A:: 0,1 % Trifluoressigsäure
   B: wie A + 80 % Acetonitril
   - Gradient:: Start - 25 % B
   55 min - 50 % B
   60 min - 100 % B
   - Absorption:: 230 nm
   - Flußrate:: 0,7 ml/min
Abb. 11: Zeitlicher Verlauf der intrazellulären cAMP-Konzentration von PC-12-Zellen nach Stimulation mit hPTH-Fragmenten. 1,5 x 10⁵ Zellen wurden mit 10⁻⁷ M hPTH-(1-33), hPTH-(1-37) und hPTH-(1-38) in Gegenwart von 10⁻⁴ M IBMX 0, 2,5, 5, 10 und 20 min lang inkubiert. Zur Kontrolle wurden Zellen 20 min lang nur mit IBMX inkubiert. Die Werte sind Mittelwerte von Dreilachansätzen +- S.D.
Abb. 12: Dosis-Wirkungs-Beziehung zwischen der Konzentration hPTH-(1-33), hPTH-(1-37) und hPTH-(1-38) und intrazellulärem cAMP-Spiegel in PC-12-Zellen nach 5 min Stimulation. 2,2 x 10⁵ PC-12-Zellen wurden 5 min lang mit jeweils 10⁻¹¹ (Doppelansatz), 10⁻¹⁰, 10⁻⁹, 10⁻⁸ und 10⁻⁷ M hPTH-(1-33), hPTH-(1-37) und hPTH-(1-38) in Dreilachansätzen in Gegenwart von 10⁻⁴ M IBMX inkubiert und die intrazelluläre cAMP-Konzentration gemessen. IBMX-Kontrollwerte ohne PTH-Fragmente: 18,0, 26,1, 20,9. Die abgebildeten Werte sind Mittelwerte +- S.D.
Abb. 13: UV-Zirkular-Dichroismus-Spektren der PTH-Fragmente hPTH-(1-33), hPTH-(1-37) und hPTH-(1-38). Im Vergleich zu anderen hPTH-Fragmenten weist das hPTH-(1-37) eine besonders prominente Struktur auf.
Abb. 14: Aufreinigung des chemisch synthetisierten hPTH-(1-37) im ersten Schritt. Hier wurde für das Rohprodukt eine Kationen-Austauscher-Chromatographie durchgeführt. Nach der Referenz erscheint das synthetische hPTH-(1-37) in dem größeren Peak bei einer Retentionszeit von 29 min (Pfeil).
   - Säule:: Parcosil PepKat 300-7, 125 x 4 mm
   - Temperatur:: 25° C
   - Elutionsmittel:: A: 5 mM NaH_{*2*}PO_{*4*}
   B: 5 mM NaH_{*2*}PO_{*4*} 1 M NaCl, pH = 3,0
   - Gradient:: Start - 5 % B
   57 min - 100 % B
   - Absorption:: 230 nm
   - Flußrate:: 1 ml/min
Abb. 15: Reversed-Phase-HPLC-Chromatographie des vorgereinigten Materials aus Abbildung 14 im semipräparativen Maßstab. Man erhält einen ab 22,50 min eluierenden Hauptpeak, der der Referenz des hPTH-(1-37) entspricht.
   - Säule:: Parcosil ProRP 300-7, C 4,100 x 20 mm
   - Temperatur:: 25° C
   - Absorption:: 230 nm
   - Elutionsmittel:: A: 0,1 % Trifluoressigsäure
   B: wie A + 80 % Acetonitril
   - Gradient:: 0 - 100 % B in 60 min
   - Flußrate:: 7 ml/min
Abb. 16: Reversed-Phase-HPLC-Chromatographie des Materials aus Abbildung 15 mit Darstellung der Endreinigung. Der Peak entsprich der Referenz von chemisch synthetisiertem hPTH-(1-37).
   - Säule:: Parcosil ProRP, 300-7, C 18, 125 x 4 mm
   - Temperatur:: 25° C
   - Absorption:: 230 nm
   - Elutionsmittel:: A: 0,1 % Trifluoressigsäure B: wie A + 80 % Acetonitril
   - Gradient:: 0 - 100 % B in 60 min
   - Flußrate:: 0,7 ml/min
Abb. 17: Plasmadesorptionsmassenspektrum des synthetischen hPTH-(1-37) aus der Präparation von Abbildung 16. Das gefundene Molekulargewicht von MG 4398,1 stimmt im Fehlerbereich < 0,1 % mit der theoretisch berechneten Masse von MG 4401,0 des Moleküls überein. Nebensequenzen sind nicht sichtbar. Der Peak bei MG 2201,5 entspricht der doppelt ionisierten Form, ebenfalls innerhalb der Fehlergrenze der Messung. Diese Massenbestimmung wurde durch Gegensequenzierung bestätigt.

### Beispiel 1

### Indirekter Nachweis der Sequenz eines zirkulierenden, biologisch aktiven PTH-Fragments

### Als Ausgangsmaterial wurde Hämofiltrat verwendet, das in großen Mengen bei der Behandlung niereninsuffizienter Patienten anfällt und alle Plasmabestandteile bis zu einer Molekülgröße von ca. 20.000 Dalton enthält.

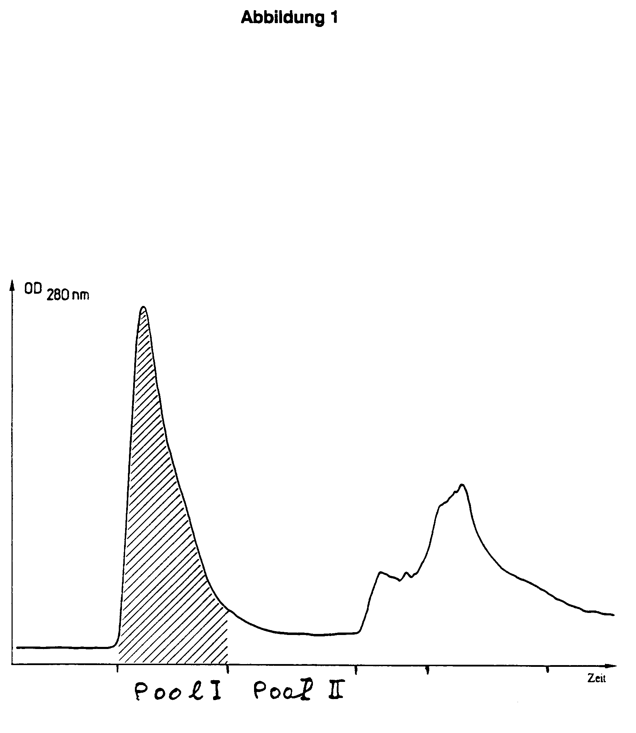

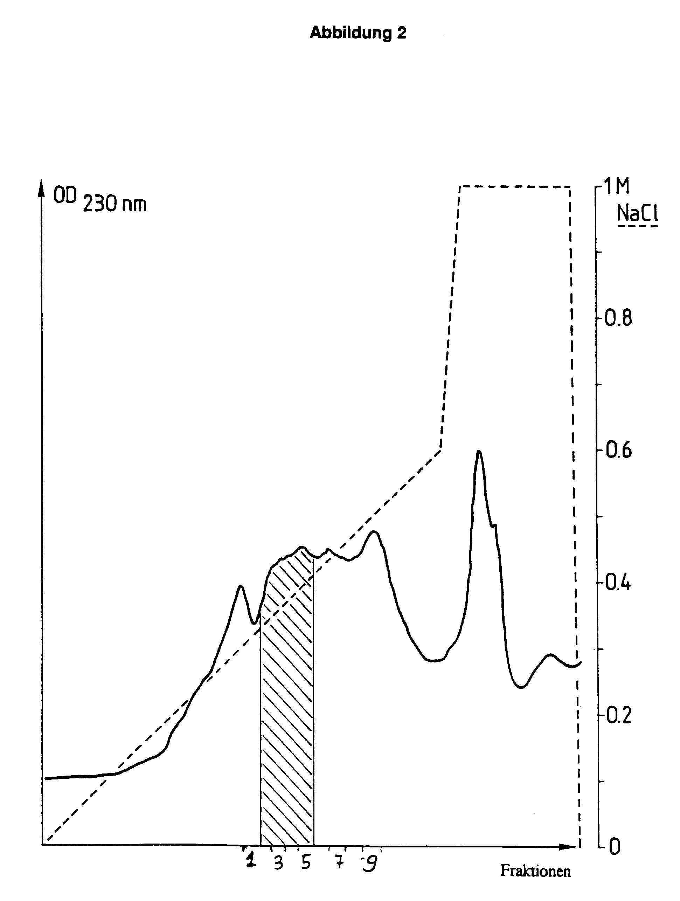

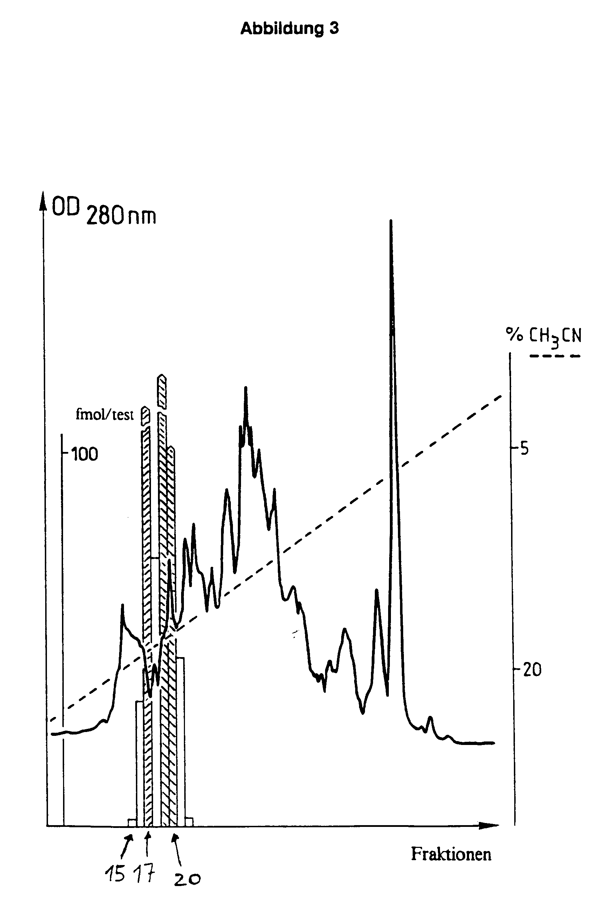

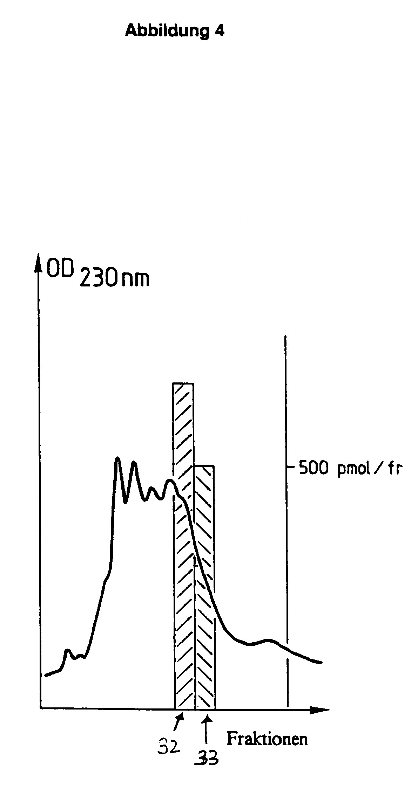

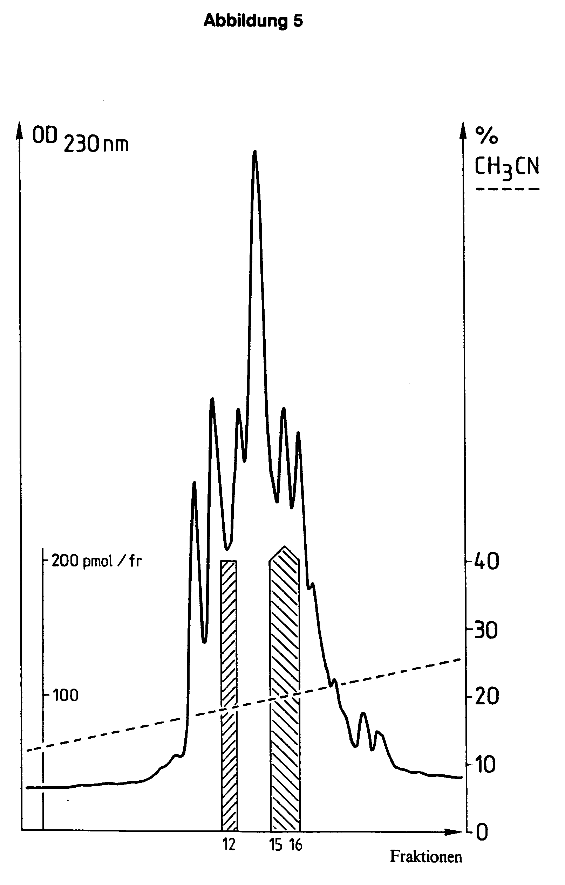

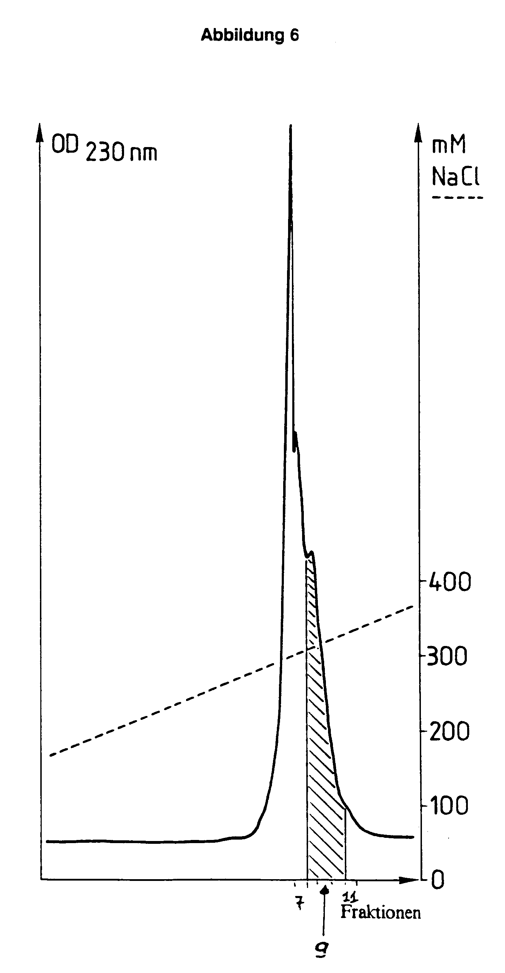

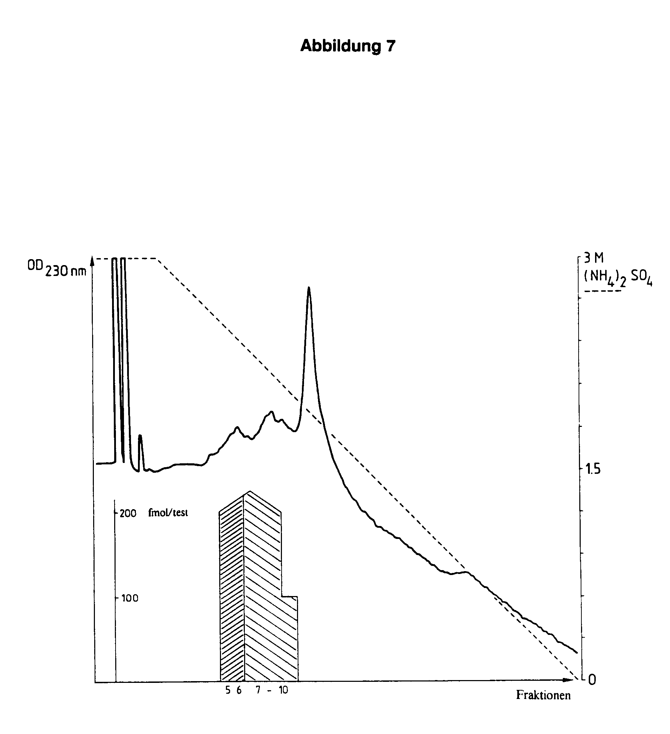

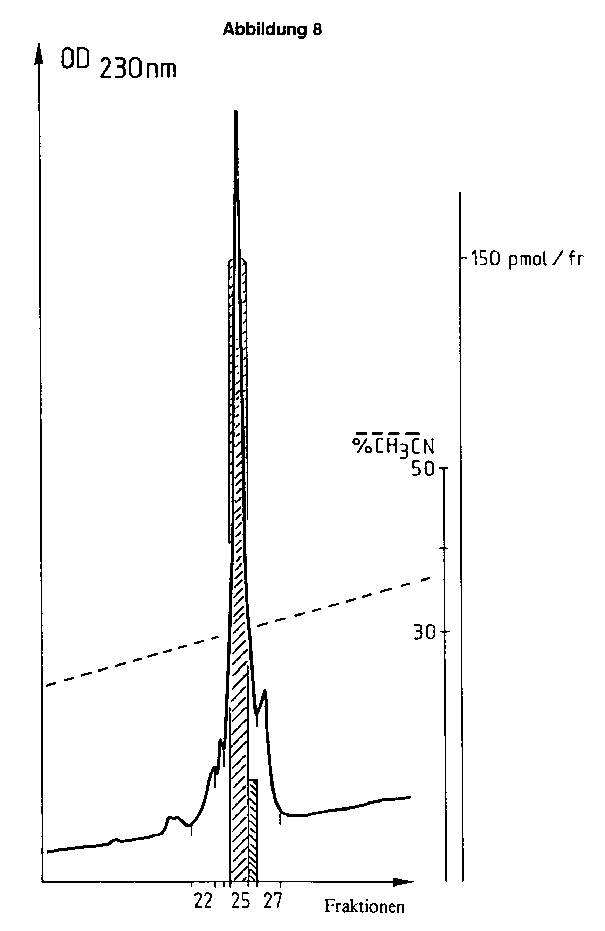

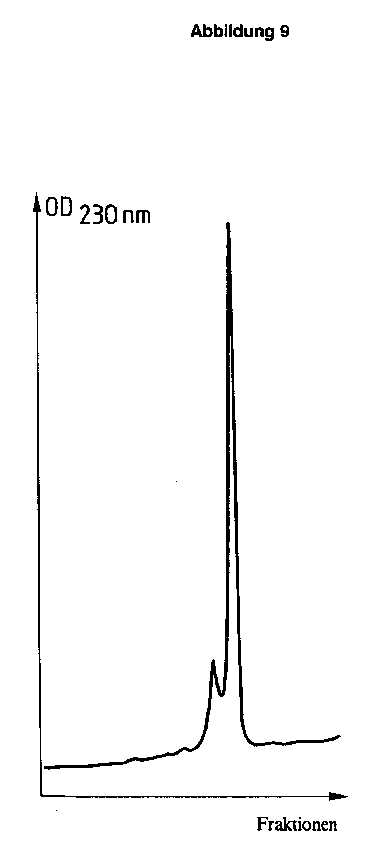

**I. Gewinnung des Rohpeptidmaterials**
   Das Hämofiltrat wurde mittels einer Hämofiltrationsanlage der Firma Sartorius unter Verwendung von Cellulosetriacetat-Filtern mit einer Ausschlußgröße von 20.000 Dalton (Typ SM 40042, Sartorius, Göttingen, BRD) gewonnen. Das Filtrat stammte von niereninsuffizienten Patienten, die sich durch Langzeit-Härmofiltration in einer stabilen Stoffwechsellage befanden. 1000 l Hämofiltrat wurden gewonnen und unmittelbar nach der Gewinnung mittels Durchlauferhitzung, Ansäuern und Zugabe von Enzyminhibtoren gegen proteolytischen Abbau geschützt. Anschließend wurde durch Alginsäureextraktion nach dem Verfahren von Forssmann der DE 36 33 797 A1 eine Rohpeptidfraktion (ca. 100 g) gewonnen.
**II. Isolierung einer Fraktion (Code 51.5) mit Immunreaktivität in mittelregional- und C-terminal-spezifischen Radioimmunoassays für Parathormon (PTH)**
   1. **Entsalzen des Rohpeptidmaterials über Sephadex G-25**
      Die Rohpeptidfraktion aus der Alginsäure-Extraktion wurde über eine mit Sephadex® G-25 (Pharmacia, Uppsala, Schweden) gefüllte Säule, die mit 1 M Essigsäure equilibriert war, bei 8° C gelchromatographisch aufgetrennt (Abb. 1).
      Die Immunreaktivität in den Pools I bis IV wurde in einem Radioimmunoassay (RIA) für humanes PTH mit mittelregionaler Spezifität [hPTH-(44-68)-RIA, Fa. Immundiagnostik, Darmstadt, BRD] gemessen. Nach den Angaben des Herstellers beträgt die untere Nachweisgrenze 6 fMol/Test des verwendeten Standards [hPTH-(44-68)]. Intra-Assay- bzw. Inter-Assay-Variationskoeffrzienten liegen bei 10 bis 13 % bzw. 12 bis 21 %. Die synthetischen hPTH-Peptide hPTH-(1-34), hPTH-(28-48) und hPTH-(64-84) zeigen keine Kreuzreaktion, die Peptide hPTH-(53-84) und hPTH-(1-84) 100 % Kreuzreaktion im hPTH-(44-68)-RIA.
      Pool I enthielt die gesamte im hPTH-(44-68)-RIA gemessene Immunreaktivität, während in den anderen Fraktionen keine Immunreaktivität nachzuweisen war.
      Pool I enthielt auch die höchste C-terminale Immunreaktivität, gemessen im hPTH-(53-84)-RIA (Fa. Immundiagnostik, Darmstadt, BRD). Der hPTH-(53-84)-RIA hat nach Herstellerangabe eine untere Nachweisgrenze von 4 fMol/Test des verwendeten Standards [hPTH-(53-84)]. Die Intra-Assay- bzw. Inter-Assay-Variationskoeffizenten liegen bei 8,3 bis 9,8 bzw. 11 bis 14 %. Die synthetischen hPTH-Peptide hPTH-(1-34), hPTH-(28-48) und hPTH-(44-68) zeigen keine Kreuzreaktion, die Peptide hPTH-(64-84) und hPTH-(1-84) 100 % Kreuzreaktion im hPTH-(53-84)-RIA.
      Pool I (schraffiert) wurde für die weitere Isolierung eingesetzt, da hier aufgrund der Messungen in zwei PTH-RIA die höchste Konzentration an C-terminalen PTH-Fragmenten auftrat.
   2. **Präparative Kationen-Austainscher-Chromatographie von Pool I**
      Eine weitere präparative Auftrennung erfolgte bei Raumtemperatur über eine Kationen-Austauschersäule (Abb. 2). Es wurden zusammengenommen ca. 6 g des Materials aus Pool I in 12 identischen Chromatographieläufen aufgetrennt und die Immunreaktivität im hPTH-(44-68)-RIA verfolgt (Abb. 2). Die in der Tabelle nicht gezeigten Fraktionen 2-5 (schraffiert) enthielten die höchste Immunreaktivität im hPTH-(44-68)-RIA und wurden für die weitere Auftrennung gepoolt. Sie enthielten auch die höchste Immunreaktivität im hPTH-(53-84)-RIA.
   3. **Semipräparative Reversed**-**Phase-Chromatographie des immunoreaktiven Pools aus der präparativen Kationen-Austauscher-Chromatographie (Stufe 2)**
      Der immunreaktive Pool (ca. 200 mg) wurde über eine semipräparative Reversed-Phase (RP)-Chromatographie bei 45° C weiter gereinigt (Abb. 3). Die Immunreaktivität im hPTH-(53-84)-RIA (Abb. 3) bzw. im hPTH-(44-68)-RIA (Abb. 3) trat in zwei unvollständig voneinander getrennten Peaks auf.
      Die Aufarbeitung des hinteren, im RIA starker reagierenden Pools wird im folgenden genauer geschildert (Abb. 4 bis 6) und führte nach Durchlaufen der Stufen 4 bis 6 zu der Fraktion 51.5, die einer Sequenzanalyse zugeführt wurde.
      Die nicht aufgeführten Fraktionen enthielten keine meßbare Immunreaktivität.
   4.
      a) **Semipräparative Kationen**-**Austauscher**-**Chromatographie des immunreaktiven Pools aus der semipräparativen RP-Chromatographie (Stufe 3)**
         Der hintere immunreaktive Pool aus der vorhergehenden RP-Chromatographie wurde über eine semipräparative Kationen-Austauschersäule bei Raumtemperatur chromatographiert (Abb. 4) und die mittelregionale und C-terminale PTH-Immunreaktivität (ohne Abb.) erschien in den Fraktionen 32-33. Diese wurden gepoolt (Schraffur) und weiterverarbeitet. Die nicht aufgeführten Fraktionen enthielten keine meßbare Immunreaktivität.
      b) **Analytische RP-Chromatographie des immunreaktiven Pools aus 4a)**
         Der immunreaktive Pool aus der vorhergehenden semipräparativen Kationen-Austauscher-Chromatographie wurde bei 45° C über eine analytische RP-Säule aufgetrennt (Abb. 5). Der hintere immunreaktive Pool mit höherer Immunreaktivität (schraffiert) wurde weiterverarbeitet. Die nicht aufgeführten Fraktionen enthielten keine meßbare Immunreaktivität.
      c) **Analytische Kationen-Austauscher-Chromatographie des immunreaktiven Pools aus 4b)**
         Der immunreaktive Pool wurde über eine analytische Kationen-Austauschersäule bei Raumtemperatur chromatographiert (Abb. 6). Die Immunreaktivität im hPTH-(44-68)-RIA ist in Abbildung 6 zu sehen (schraffiert), die Messung im hPTH-(53-84)-RIA ergab wiederum das gleiche Bild. Die nicht aufgeführten Fraktionen enthielten keine meßbare Immunreaktivität. Die Fraktionen mit der höchsten Immunreaktivität wurden gepoolt (Schraffur) und weiterverarbeitet.
   5. **Hydrophobe Interaktionschromatographie des immunreaktiven Pools aus 4c)**
      Der immunreaktive Pool aus 4c) wurde mit Hilfe der hydrophoben Interaktionschromatographie bei Raumtemperatur weiter aufgetrennt (Abb. 7) und die Immunreaktivität im hPTH-(44-68)-RIA gemessen (Abb. 7). Die nicht aufgeführten Fraktionen enthielten keine meßbare Immunreaktivität. Die Fraktionen mit höchster Immunreaktivität (schraffiert) wurden gepoolt und der Endreinigung zugeführt.
   6.
      a) **Erste analytische RP-Chromatographie**
         Der immunreaktive Pool aus 5) wurde über eine RP-Säule bei 25° C chromatographiert und die Immunreaktivität mit dem hPTH-(44-68)-RIA verfolgt (Abb. 8). Die schraffiert angegebenen Fraktionen wurden rechromatographiert.
      b) **Zweite analytische RP-Chromatographie**
         Der immunreaktive Pool aus 6a) wurde nochmals über eine RP-Säule gegeben (Abb. 9). Mit Ausnahme der Temperatur, die bei dieser Chromatographie 45° C betrug, wurde unter den gleichen Bedingungen wie bei Stufe 6a) chromatographiert. Das Material des höheren Peaks mit der Code-Nr. 51.5 wurde sequenziert.
   **III. Isolierung einer zweiten Fraktion (Code 54.5) mit Immunreaktivität in mittelregional- und C-terminal-spezifischen Radioimmunassays für Parathormon (PTH)**
      Bei der Isolierung der Fraktion 51.5 traten bei der semipräparativen RP-Chromatographie (Stufe 3) 2 Pools mit Immunreaktivität auf. Die Aufarbeitung des vorderen Pools aus Stufe 3 erfolgte in identischer Weise wie für den hinteren Pool unter II. 4 bis 6 beschrieben. In Stufe 4b) wurden wiederum zwei immunreaktive Peaks gesehen, wobei in diesem Fall der vordere Peak überwog und weiterverarbeitet wurde. Nach Durchlaufen der Stufen 4c) bis 6b) wurde eine Fraktion mit dem Code 54.5 erhalten, die ebenfalls einer Sequenzanalyse zugeführt wurde.
   **IV. Sequenzierung der aus Hämofiltrat isolierten PTH-Peptide mit Immunreaktivität in mittelregional- und C-terminal-spezifischen Radioimmunoassays für Parathormon (PTH)**
      Die Fraktionen 51.5 und 54.5, deren Isolierung aus Hämofiltrat unter II und III beschrieben wurde, wurden mit Hilfe des Gasphasensequenators Typ 470 A (Applied Biosystems) sequenziert. Der EDMAN-Abbau (*Edman und Begg, Eur. J. Biochem. 1, 80-91, 1967*) erfolgte mit dem Standardprogramm PTHRUN. Die Analyse der derivatisierten Aminosäuren wurde im on-line-Verfahren über einen ABI 120 A Chromatographen unter Verwendung des Standardprotokolls ABI durchgeführt. Für die Fraktion 51.5 konnte eine Sequenz von 39 Aminosäuren bestimmt werden, weitere 5 Aminosäure-Reste konnten nur mit Vorbehalt ermittelt werden. Die gefundene Sequenz entspricht dem Abschnitt (38-76) des humanen PTH (*Keutmann et al., Biochemistry 17, 5723-5729, 1978)*.
      Für die Fraktion 54.5 konnte eine Sequenz von 16 Aminosäuren bestimmt werden. Die gefundene Sequenz entspricht dem humanen PTH-(38-53).
      Aus dem menschlichen Hämofiltrat konnten zwei Fraktionen isoliert werden, die beide sowohl in einem mittelregionalen als auch in einem C-terminalen PTH-RIA detektiert wurden. Beide Peptide beginnen mit der aminoterminalen Sequenz Gly-Ala-Pro-Leu-Ala-Pro-Arg-usw., d.h. beide Peptide beginnen mit der Aminosäure 38 des hPTH-Moleküls. Die letzte C-terminale Aminosäure kann für beide gefundenen Peptide nicht mit Sicherheit angegeben werden. Im Fall der Fraktion 51.5 konnte fast bis zur Position 84 durchsequenziert werden. Das andere Peptid, von dem wegen der geringeren zur Verfügung stehenden Menge nur 16 Positionen durch Sequenzanalyse bestimmt werden konnten, hat mindestens eine Sequenzlänge von 30 Aminosäuren, da es sonst nicht im C-terminalen RIA detektiert würde. C-terminale PTH-Peptide mit einem anderslautenden N-terminalen Sequenzanfang konnten nicht isoliert werden. Diese Befunde lassen den Schluß zu, daß das hPTH-(1-84) zwischen den Aminosäuren 37 und 38 gespalten wird, und daß dabei das biologisch aktive PTH-Fragment hPTH-(1-37) entsteht.

### Beispiel 2

### Synthese des humanen Parathormon-Peptids hPTH-(1-37)

1. **Synthese des kieselgurverstärkten Fmoc-Leu-Harzes**

   Das Fmoc-Aminosäureanhydrid (5 Äquivalente) wurde in einem Minimalvolumen N,N-Dimethylformamid (DMF) gelöst und in einem Rundkolben zu dem Hydroxyethylphenoxyacetyl-norleucin-Harz hinzugefügt. 4-Dimethylaminopyridin. (1 Äquivalent) wurde ebenfalls in einem Minimalvolumen DMF gelöst und in den Rundkolben gegeben. Nach 1 Stunde Reaktionszeit wurde der Überschuß der Reagentien durch Filtration entfernt und das Harz auf einem Filter gründlich gewaschen.
2. **Strategie der Synthese des humanen Parathormon-Peptids hPTH-(1-37)**
   Für die Synthese des Peptids mit der Formel 
Ser-Val-Ser-Glu-Ile-Gln-Leu-Met-His-Asn-Leu-Gly-Lys-His-Leu-Asn-Ser-Met-Glu-
Arg-Val-Glu-Trp-Leu-Arg-Lys-Lys-Leu-Gln-Asp-Val-His-Asn-Phe-Val-Ala-Leu
 wurde die Durchflußmethode (*Atherton und Sheppard, Solid phase peptide synthesis. IRL Press, Oxford*, *1989*) angewendet. Die genannte Peptidsequenz wurde mit Hilfe einer automatischen Peptidsynthese-Apparatur (Milligen 9050) unter Verwendung der Fmoc-Pentafluorophenylester (OPfp) synthetisiert. Folgende Fmoc-Aminosäuren-OPfp wurden im Überschuß (4 Äquivalente) eingesetzt (verwendet wurden jeweils die Derivate der L-Aminosäuren):
   - Fmoc-Ala-OPfp: Fmoc-Ala
   - Fmoc-Asp(OBu^{t})-OPfp: Fmoc-Asp(OBu^{t})
   - Fmoc-Met-OPfp: Fmoc-Met
   - Fmoc-Glu(OBu^{t})-OPfp: Fmoc-Glu(OBu^{t})
   - Fmoc-His(Trt)-OPfp: Fmoc-His(Trt)
   - Fmoc-Leu-OPfp: Fmoc-Leu
   - Fmoc-Arg(Mtr)-OPfp: Fmoc-Arg(Mtr)
   - Fmoc-Trp-OPfp: Fmoc-Trp Fmoc-Lys(BOC)-OPfp Fmoc-Lys(BOC)
   - Fmoc-Ile-OPfp: Fmoc-Ile
   - Fmoc-Phe-OPfp: Fmoc-Phe
   - Fmoc-Gly-OPfp: Fmoc-Gly
   - Fmoc-Asn-OPfp: Fmoc-Asn(Trt)
   - Fmoc-Gln-OPfp: Fmoc-Gln(Trt)
   - Fmoc-Val-OPfp: Fmoc-Val
   - Fmoc-Ser(Bu^{t})-ODhbt: Fmoc-Ser(Bu^{t})
   Die Synthese kann auch mit Hilfe von *in situ* durch Zugabe von TBTP [O-(1H-Benzotriazol-1-yl)-N,N,N',N'tetramethyluronium-tetrafluoroborat] gebildeten Hydroxybenxotriazolestern durchgeführt werden.
   Das Peptid wurde vom Trägerharz durch Zugabe einer Mischung von Trifluoressigsäure-Anisol-Ethandithiol-Phenol 94:2:2:2 (v/v/v/w) abgespalten und mit Ether gefällt. Das HPLC-gereinigte Peptid wurde mit Hilfe von Aminosäureanalyse, analytischer HPLC und Aminosäuresequenzanalyse charakterisert.
3. **Praktische Durchführung der Synthese des humanen Parathormon-Peptids hPTH-(1-37)**
   Die Synthese von 
Ser-Val-Ser-Glu-Ile-Gln-Leu-Met-His-Asn-Leu-Gly-Lys-His-Leu-
Asn-Ser-Met-Glu-Arg-Val-Glu-Trp-Leu-Arg-Lys-Lys-Leu-Gln-Asp-
Val-His-Asn-Phe-Val-Ala-Leu
 (I) hPTH-(1-37)
   wurde mit dem automatischen 9050 PepSynthesizer (Programm Version 1.3), MilliGen/Biosearch, nach der continuous flow-Methode durchgeführt. Fmoc-Aminosäurepentafluorophenylester waren L-konfiguriert und in 0,8 mmol-Portionen verwendet. Alle zur Synthese notwendigen Reagenzien wurden von MilliGen/Biosearch bezogen: N,N-Dimethylformamid, 20 % Piperidin in N,N-Dimethylformamid und 1-Hydroxybenzotriazol. Die L-Aminosäurederivate wurden in vierfachem Überschuß eingesetzt. Die terminalen Aminogruppen waren Fmoc-geschützt. Asparagin- und Glutaminsäure wurden als N^{W}-tert-butylester, Tyrosin und Serin als tert-butylether, Histidin und Lysin als N^{W}-Boc-Verbindungen und Arg als N^{G}-2,2,5,7,8-pentamethylchroman-6-sulfonyl(Pmc)-Derivat eingesetzt. Die Synthese wurde ausgehend vom Kieselgur-Harz Fmoc-Leu-Pepsyn KA (MilliGen/Biosearch), also mit trägergebundener C-terminaler Aminosäure (0,091 meq Leu/g, 1,60 g) durchgeführt. Die Acylierung von Fmoc-Arg(Pmc)-OH wurde unter Anwesenheit von O-(1H-Benzotriazol-1-yl)-N,N,N',N'-tetramethyluronium-tetrafluoroborat (TBTU), 1-Hydroxybenzotriazol und Diisopropylethylamin durchgeführt. Folgender Synthesecyclus wurde verwendet: Fmoc-Abspaltung mit 20 % Piperidin in DMF (7 min), waschen mit DMF (12 min), Acylierung (30 min; bei Fmoc-Val-OPfp 45 min), und waschen mit DMF (8 min). Die fortschreitende Synthese wurde durch kontinuierliche UV-Detektion verfolgt. Die Synthese wurde mit der Abspaltung des N-terminalen Fmoc-Restes abgeschlossen. Das harzgebundene Peptid wurde dreimal mit je 50 ml Isopropanol, Eisessig, Isopropanol und Diethylether gewaschen und getrocknet.

Die Abspaltung vom Harz erfolgte mit Trifluoressigsäure/Phenol/Ethandithiol/Thioanisol/Wasser 10 : 0,75 : 0,25 : 0,5 : 0,5 (v/w/v/v/v, 5 ml). Die Lösung wird *in vacuo* eingeengt und das Produkt durch Zugabe von Diethylether ausgefällt. Das erhaltene Rohpeptid wird mehrere Male mit Diethylether gewaschen und getrocknet. Aus 240 mg Peptid-Harz werden so 65 mg Rohpeptid erhalten.

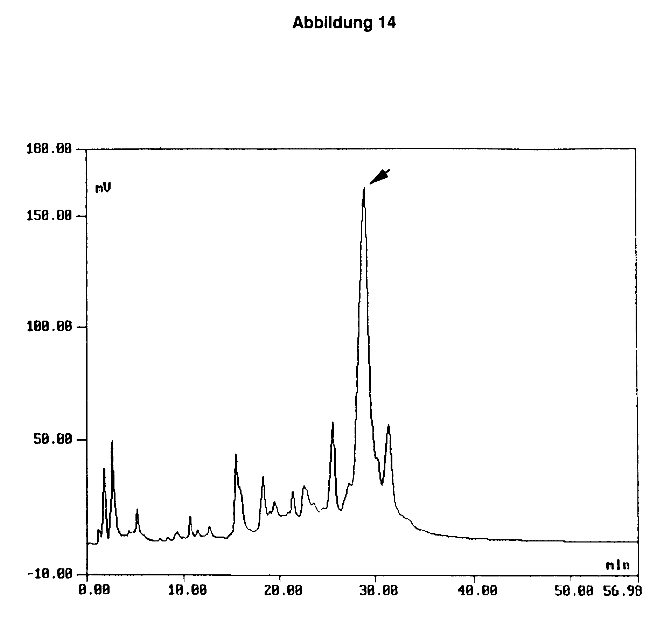

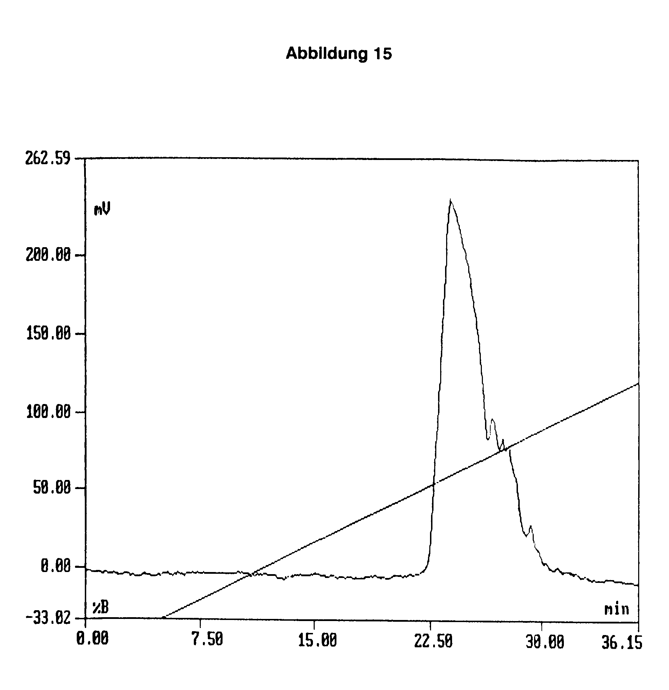

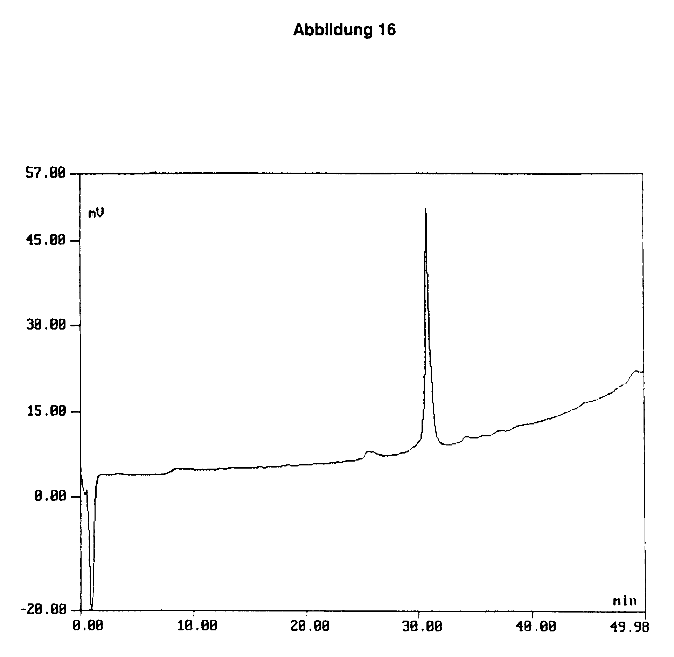

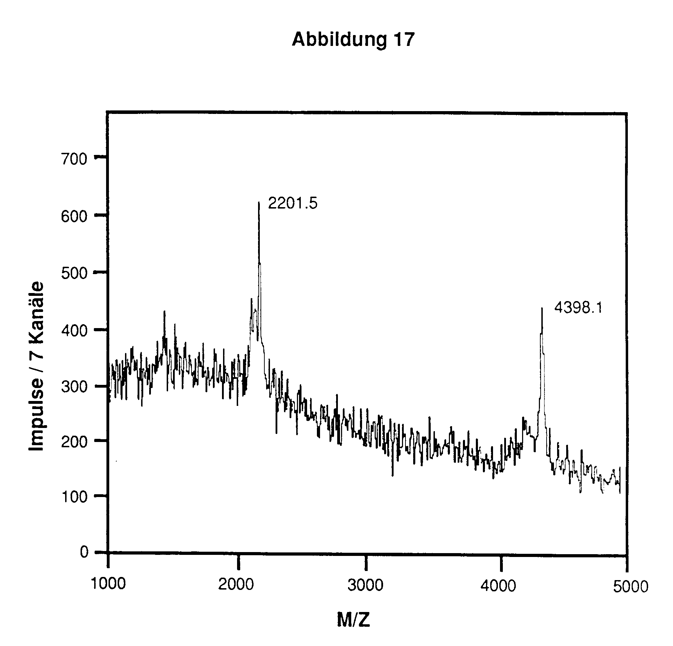

Die Reinigung erfolgte unter Benutzung eines in unabhängiger Synthese hergestellten Standards von hPTH-(1-37), dessen korrekte Sequenz durch MS (Massenspektrometrie) und Sequenzanalyse bestätigt war. Im ersten Schritt wurde das deblockierte Rohmaterial mit einer Kationen-Austauscher-HPLC gereinigt (Parcosil PepKat 2 x 10 cm, 300 Å, 7 », Flußrate 9 ml/min, 230 nm, Elutionsmittel A= 5 mM NaH_{*2*}PO_{*4*}; B= 5 mM NaH_{*2*}PO_{*4*} + 1 M NaCl, Gradient: 5 % → 100% in 60 min), wobei der Hauptpeak entsprechend der Referenzsubstanz bei 28,17 min erscheint (Abb. 14). Im zweiten Reinigungschritt bei gleichzeitiger RP-HPLC zur Entsalzung (Parcosil ProRP C4 2 x 10 cm, 300 Å, 7 », Flußrate 7 ml/min, 230 nm, Elutionsmittel A= 0,1 % Trfluoressigsäure in Wasser; B= 0.1 % Trfluoressigsäure in Acetonitril/Wasser 4:1, Gradient: 0 % → 100 % B in 60 min). Ausbeute: 6,6 mg (1,5 mmol, 14,8 %) wurde ein Peak bei 30,80 min erhalten (Abb. 15), der in einer analytischen RP-HPLC mit C 18-Material einen scharfen einheitlichen Peak ergibt (Abb. 16).
Die Identität des synthetisierten Materials mit der vorgegebenen Primärstruktur von PTH-(1-37) wurde durch MS (Plasmadesorptionsmethode, Bio-Ion, Applied Biosystems) (Abb. 17) und Gegensequenzierung in einem Gassphasensequenator (Modell 470, Applied Biosystems) nachgewiesen. Die biologische Aktivität des synthetischen PTH-(1-37 wurde im Funktionstest durch die differentielle Muskelkontraktion von Pulmonalarterien gegenüber der Arteria renalis nachgewiesen: dieser zeigte, daß das synthetische Material eine korrekte biologische Aktivität aufweist.

### Beispiel 3

### Nachweis des biologisch aktiven, zirkulierenden humanen PTH als hPTH-(1-37)

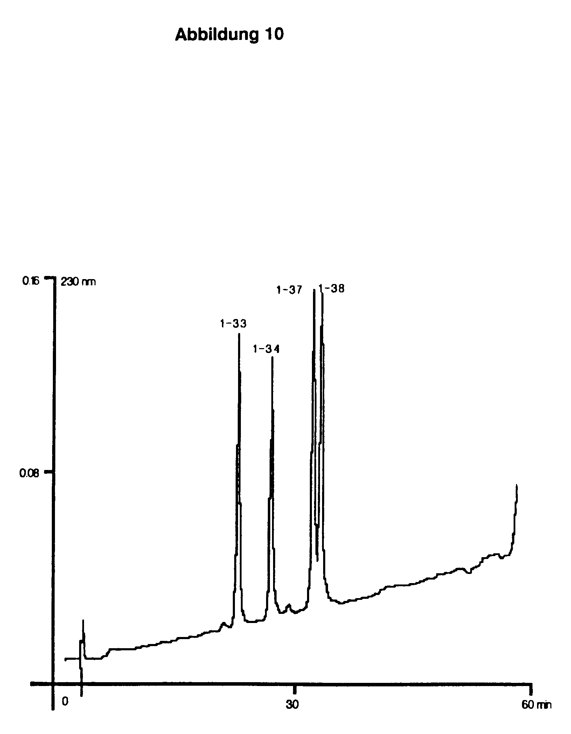

Wie in Beispiel 1 beschrieben, wurde Hämofiltrat chromatographisch aufgearbeitet, wobei das synthetische hPTH-(1-37) gemäß Beispiel 2 als Referenzsubstanz eingesetzt wurde (Abb. 10). Auf diese Weise konnte aus dem Hämofiltrat eine Fraktion nachgewiesen werden, die Immunreaktivität mit aminoregionaler Spezifität im hPTH-Radioimmunassay zeigte. Diese Fraktion wurde in gleicher Weise wie in Beispiel 1 gereinigt und als hPTH-(1-37) charakterisiert. Dabei wurde nachgewiesen, daß mit der verwendeten Parcosil-RP-Säule hPTH-(1-37) eindeutig von weiteren PTH-Fragmenten unterschieden werden kann (Abb. 10), nämlich hPTH-(1-33), hPTH-(1-34) und hPTH-(1-38).

### Beispiel 4

### Funktionelle Analyse von hPTH-(1-37) zum Nachweis seiner biologischen Aktivität

Veränderung des intrazellulären Spiegels von cyclischem 3',5'-Adenosinmonophosphat (cAMP) bei Phäochromozytomzellen (PC-12) der Ratte nach Applikation von humanen Parathormon-Peptiden hPTH-(1-33), hPTH-(1-37) und hPTH-(1-38).

In Vorversuchen wurde gezeigt, daß eine Phäochromozytomlinie der Ratte (PC-12) auf Zugabe von PTH mit einer Erhöhung des intrazellulären cAMP-Spiegels reagiert. Daraufhin wurde die Wirkung des synthetischen hPTH-Peptides (1-37) mit der Wirksamkeit anderer N-terminaler PTH-Fragmente [hPTH-(1-33) und hPTH-(1-38)] in diesem Zell-System verglichen.

Es wurde eine PC-12 Zell-Linie verwendet. Die Zellen wurden in RPMI-Medium 1640 (Gibco) mit 10 ml/L L-Glutamin 200 M (Gibco), 10 % Pferdeserum, 5 % FCS und 1 % Penicillin/Streptomycin kultiviert. Als Phosphodiesterase-Hemmer wurde 4-Isobutyl-1-methyl-xanthin (IBMX) von Sigma verwendet. Es wurden Kulturschalen mit 24 Löchern (Durchmesser 16 mm) (Costar), beschichtet mit Poly-L-Lysin, verwendet. Die Beschichtung erfolgte mit steril filtrierter Poly-L-Lysin-Lösung (100 mg/L) für 1 Stunde bei 37° C.

Es wurden die folgenden hPTH-Peptide untersucht:
hPTH-(1-33) (MW. 3971,2; Peptidgehalt 76,9 %), hPTH-(1-37) (MW. 4401,0;
Peptidgehalt 74,5 %) und hPTH-(1-38) (MW. 4458,0; Peptidgehalt 81,8 %).

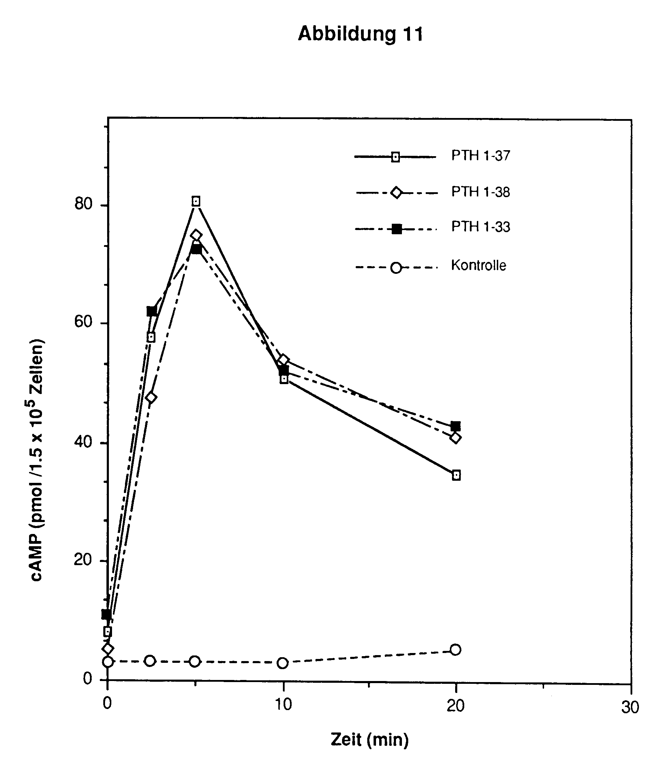

Die Zellen wurden nach Ausbildung eines dichten Zellrasens (nach ca. 4-8 Tagen) verwendet. Die Inkubation erfolgte mit 1 ml Zellmedium bei Raumtemperatur. Die PTH-Peptide wurden unmittelbar vor Versuchsbeginn mit Zellmedium verdünnt. Im ersten Versuchsansatz wurden 1,5 x 10⁵ Zellen mit 10^{-*7*} M hPTH(1-33), hPTH-(1-37) und hPTH-(1-38) in Gegenwart von 10^{-*4*} IBMX 0, 2,5, 5, 10 und 20 Minuten lang inkubiert. Zur Kontrolle wurden Zellen 20 Minuten lang nur mit IBMX inkubiert. Die Werte sind Mittelwerte von Dreifachansätzen ± S.D. (vgl. Abb. 11).

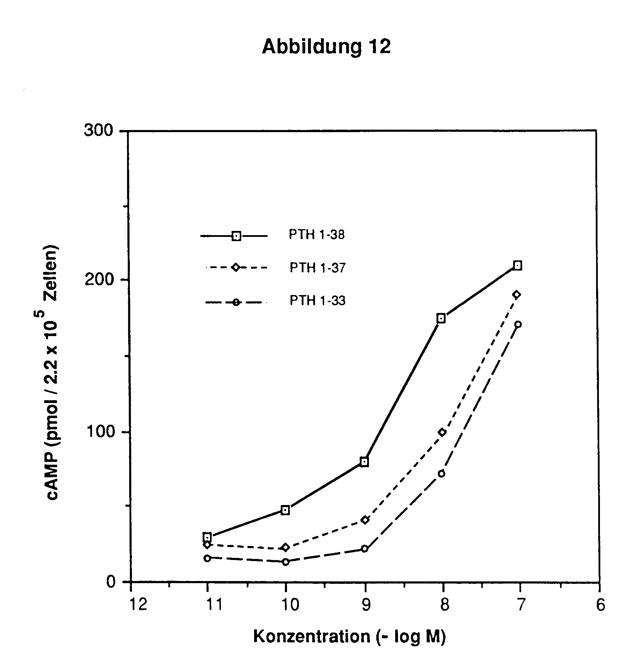

In einem zweiten Versuchsansatz wurden 2,2 x 10^{*5*} PC-12-Zellen 5 Minuten lang mit jeweils 10^{-*11*} (Doppelansatz), 10^{-*10*} 10^{-*9*}*,* 10^{-*8*} und 10^{-*7*} M hPTH-(1-33), hPTH-(1-37) und hPTH-(1-38) in Dreifachansätzen in Gegenwart von 10^{-*4*} M IBMX inkubiert und die intrazelluläre cAMP-Konzentration gemessen. IBMX-Kontrollwerte ohne PTH: 18,0, 26,1, 20,9. Die abgebildeten Werte sind Mittelwerte ± S.D. (vgl. Abb. 12).

Der Zusatz von PTH und IBMX erfolgte gleichzeitig. Nach Absaugen des Mediums wurde die Reaktion durch Zusatz von 1 ml 99 % Ethanol gestoppt. Die Zellen wurden abgekratzt und mit dem Ethanol in Plastikröhrchen (Greiner) überführt. Die Kulturschale wurde mit 66 % Ethanol nachgespült, die Überstände vereinigt und zentrifugiert. Der Überstand wurde im Wasserbad (50° C) unter Stickstoffbegasung eingedampft. Die Konzentration des cAMP wurde mit Hilfe eines Radioimmunoassay-Kit (NEN) bestimmt. Die Messung erfolgte nach den vom Hersteller angegebenen Vorschriften. Die eingesetzten Mengen an cAMP-Standard, cAMP-Antiserum-Komplex, cAMP ^{*125*}I-Tracer-Konzentrat, cAMP-Trägerserum und cAMP-Präzipitator betrugen 50 % der vom Hersteller angegebenen Menge.

Die Ergebnisse zeigen, daß signifikante Unterschiede in der Wirksamkeit des hPTH-(1-37) im Vergleich zu anderen, nicht endogen vorkommenden PTH-Peptiden in dem untersuchten Zellsystem bestehen. Dieses Ergebnis laßt auf Unterschiede in der Konformation dieser Peptide schließen, die sich, wie hier gezeigt, auf die Aktivierung des PTH-RezeptorAdenylatcyclase-Systems auswirken, darüber hinaus aber auch die Antigenität beeinflussen.

### Beispiel 5

### Untersuchungen zur Sekundärstruktur der Parathormon-Fragmente hPTH-(1-33), hPTH-(1-37) und hPTH-(1-38) durch Zirkular-Dichroismus

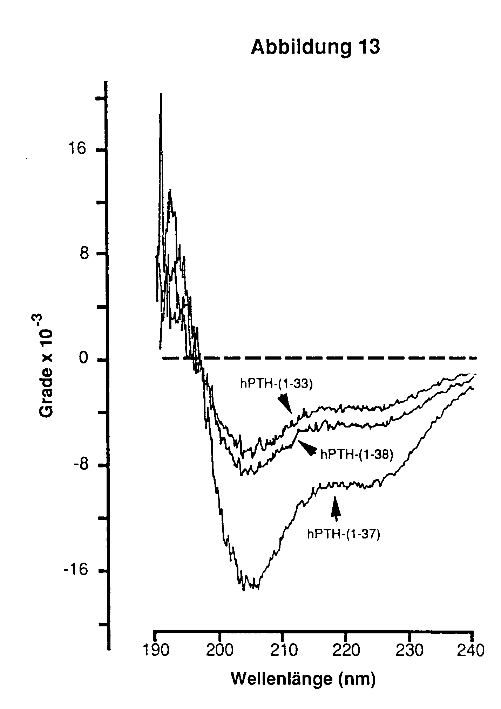

Die Messung des Zirkular-Dichroismus-Spektrums erfolgte mit einem Jasco J-500 Automatic Recording Spectropolarimeter gekoppelt mit einem Jasco DP-500 Data Processor. Zur Bestimmung der Sekundärstruktur wurde im Spektralbereich 190-240 nm, bei Raumtemperatur, in einer ausgewählten Quarzküvette, bei einem "light path" von 1 mm, gemessen. Die eingesetzte Parathormonkonzentration betrug 50 »g/ml hPTH-(1-33), 50 »g/ml hPTH-(137) und 50 »g/ml hPTH-(1-38). Zur Lösung wurde 10 mM TRIS-HCl-Puffer pH 7,5 verwendet. (Übrige Meßbedingungen: Empfindlichkeit, 2 milli Grad/cm; Zeitkonstante, 2 sek. Schreibergeschwindigkeit, 5 nm/min.; Wellenlängenexpansion, 5 nm/cm.) Die Kurven in der Abbildung 13 für hPTH-(1-33), hPTH-(1-37) und hPTH-(1-38) sind Mittelwerte der Signale von 4 aufeinander folgenden Messungen abzüglich der Basislinienwerte.

Die Auswertung zur Ermittlung der Sekundärstruktur erfolgte nach dem von *Reed* und *Kinzel* (*Biochemistry 23, 1357-1362, 1984*) beschrieben Verfahren. Aus den vorhandenen Daten wurde für die Sekundärstruktur des hPTH-(1-38) ein α-Helix-Anteil von etwa 27 % errechnet. Das um eine Aminosäure kürzere PTH-Fragment [hPTH-(1-37)] dagegen weist einen deutlich höheren Anteil an α-Helix-Struktur von etwa 40-45 % auf. Der Anteil an α-Helix beim hPTH-(1-33) ist wieder ähnlich niedrig wie beim hPTH- (1-38).

Aus den Untersuchungen folgt, daß bedingt durch die erheblichen Unterschiede in der Sekundärstruktur die Peptid-Rezeptor-Interaktionen und Immunoepitop-Eigenschaften des PTH-Fragments hPTH-(1-37) gegenüber den übrigen Fragmenten deutlich verschieden sein müssen.

### Beispiel 6

### Untersuchungen zur biologischen Aktivität von hPTH-(1-37) auf die glatte Muskulatur der Lungengefasse und des Corpus cavernosum (männliches Genitalorgan).

Die glatte Muskulatur der Lungengefasse und des Corpus cavernosum wurden aus menschlichen Operationspräparaten und bei Kaninchen gewonnen und in einem geeigneten Organbadsystem zur Messung der Kraftentwicklung eingespannt und untersucht. Es wurde festgestellt, daß diese Muskulatur gegenüber anderen glatten Muskeln des menschlichen Organismus nach Vorkontraktion mit verschiedenen kontraktionsaktivierenden Stoffen durch Zugabe des hPTH-(1-37) in Konzentrationen im Bereich von 10^{-*9*} Mol eine deutliche Relaxation zeigt, was beweist, daß das hPTH-(1-37) für die Vasorelaxation der Lungengefässe und die Durchblutungsaktivierung des männlichen Gliedes ein besonders geeignetes Peptid darstellt.

## Patentansprüche

1. hPTH-Fragment-(1.37) mit der Aminosäurensequenz 
Ser - Val - Ser - Glu - Ile -
Gln - Leu - Mei - His - Asn - Leu - Glv - Lvs - His - Leu - Asn - Ser - Mei - Glu -
Arg - Val - Glu - Trp - Leu - Arg - Lvs - Lvs - Leu - Gln - Asp - Val - His - Asn -
Phe - Val - Ala - Leu
 sowie seine natürlichen und pharmakologisch verträglichen Derivate, insbesondere amidierte, acetylierte, phosphorylierte und glycosylierte hPTH-(1-37)-Derivate.

2. Verfahren zur Herstellung des hPTH-Fragmentes-(1-37) oder seiner Derivate nach Anspruch 1 dadurch gekennzeichnet, daß dieses über eine prokaryontische oder eine eukaryontische Expression hergestellt und chromatographisch gereinigt wird.

3. Verfahren zur Herstellung des hPTH-Fragmentes-(1-37) oder seiner Derivate nach Anspruch 1 dadurch gekennzeichnet, daß man es aus menschlichem Blut über übliche Chromatographie-Verfahren in bekannter Weise isoliert.

4. Verfahren zur Herstellung des hPTH-Fragmentes-(1-37) oder seiner Derivate nach Anspruch 1 dadurch gekennzeichnet, daß man das hPTH-Fragment-(1-37) durch die üblichen Verfahren der Festphasen- und Flüssigphasen-Synthese aus den geschützten Aminosäuren, die in der ausgegebenen Sequenz enthalten sind, herstellt, deblockiert und es mit den gängigen Chromatographie-Verfahren reinigt.

5. Arzneimittel, enthaltend das humane, zirkulierende Nebenschilddrüsenhormon nach Anspruch 1, das hPTH-Fragment-(1-37) als aktiven. Wirkstoff neben üblichen Hilfs- und Zusatzstoffen.

6. Verwendung des hPTH-Fragmentes-(1-37) sowie seiner natürlichen und pharmakologisch vertraglichen Derivate nach Anspruch 1 zur Herstellung eines Arzneimittels zur Behandlung von Erkrankungen der Nebenschilddrüse, insbesondere bei deren Unterfunktion (Hypoparathreodismus).

7. Verwendung des hPTH-Fragmentes-(1-37) sowie seiner natürlichen und pharmakologisch vertraglichen Derivate nach Anspruch 1 zur Herstellung eines Arzneimittels zur Behandlung von degenerativen Knochenerkrankungen, insbesondere der Osteoporose.

8. Verwendung des hPTH-Fragmentes-(1-37) sowie seiner natürlichen und pharmakologisch vertraglichen Derivate nach Anspruch 1 zur Herstellung eines Arzneimittels zur Behandlung von Knochenfrakturen in der Heilungsphase.

9. Verwendung des hPTH-Fragmentes-(1-37) sowie seiner natürlichen und pharmakologisch verträglichen Derivate nach Anspruch 1 zur Herstellung eines Arzneimittels zur Behandlung von Kreislauferkrankungen, die mit Erhöhung des Blutdruckes einhergehen, insbesonders der essentiellen Hypertonie.

10. Verwendung des hPTH-Fragmentes-(1-37) sowie seiner natürlichen und pharmakologisch verträglichen Derivate nach Anspruch 1 zur Herstellung eines Arzneimittels zur Behandlung von Lungenerkrankungen, die mit Erhöhung des Blutdruckes im kleinen Kreislauf einhergehen, insbesondere der primären pulmonalen Hypertonie.

11. Verwendung des hPTH-Fragmentes-(1-37) sowie seiner natürlichen und pharmakologisch verträglichen Derivate nach Anspruch 1 zur Herstellung eines Arzneimittels zur Behandlung von Nierenerkranungen, die mit Störungen der Elektrolytausscheidung einhergehen, insbesondere bei der akuten Niereninsuffizienz sowie Phosphat- und Calciumausscheidungsstörungen.

12. Verwendung des hPTH-Fragmentes-(1-37) sowie seiner natürlichen und pharmakologisch vertraglichen Derivate nach Anspruch 1 zur Herstellung eines Arzneimittels zur Behandlung der männlichen Impotenz.

13. Verwendung des hPTH-Fragmentes-(1-37) nach Anspruch 12 in galenisch geeigneten, pharmazeutischen Zubereitungen, insbesondere in injizierbaren Lösungen, allein oder kombiniert mit anderen Peptiden wie Cardiodilatin/Urodilatin und/oder Calcitonin-Gene-Related-Peptide (CGRP) für die phasenweise Behandlung in der Fertilisationspflege und Impotenz, wobei das Peptid hPTH-(1-37) in Einzeldosen zwischen 1 und 10 Mikrogramm vorzugsweise in das männliche Glied appliziert wird.

14. Verwendung des hPTH-Fragmentes-(1-37) sowie seiner natürlichen und pharmakologisch verträglichen Derivate nach Anspruch 1 zur Herstellung eines Arzneimittels zur Diagnose von Erkrankungen, insbesondere nach irgendeinem der Ansprüche 6 - 13, in dem spezifische Antikörper gegen das synthetische Peptid hergestellt werden und die Blutkonzentration von hPTH-(1-37) über Immuntests gemessen wird.

15. Verwendung des hPTH-Fragmentes-(1-37) sowie seiner natürlichen und pharmakologisch vertraglichen Derivate nach Anspruch 1 zur Herstellung eines Arzneimittels in verschiedenen galenischen Applikationsformen, insbesondere als Lyophilisat.

16. Verwendung nach Anspruch 15, wobei die galenische Applikationsform, insbesondere die lyophilisierte, mit Mannit aufgenommene Form in sterilen Ampullen zur Auflösung in physiologischer Kochsalzlösung und/oder Infusionslösungen zur wiederholten Einzelinjektion und/oder Dauerinfusion in Mengen von 300 Mikrogramm bis 30 Milligramm reines hPTH-(1-37) pro Therapie-Einheit enthalt.

## Claims

1. The hPTH fragment-(1-37) comprising the amino acid sequence 
Ser - Val - Ser - Glu - Ile - Gln - Leu - Met - His - Asn - Leu - Gly - Lys
- His - Leu - Asn - Ser - Met - Glu - Arg - Val - Glu - Trp - Leu - Arg -
Lys - Lys - Leu - Gln - Asp - Val - His - Asn - Phe - Val - Ala - Leu
 and its natural and pharmacologically compatible derivatives, especially amidated, acetylated, phosphorylated and glycosylated hPTH-(1-37) derivatives.

2. A process for the preparation of the hPTH fragment-(1-37) or its derivatives according to claim 1, characterized in that said fragment is prepared via a prokaryotic or an eukaryotic expression and is purified by means of chromatographical procedures.

3. A process for the preparation of the hPTH fragment-(1-37) or its derivatives according to claim 1, characterized in that said fragment is isolated from human blood in a *per se* known manner by conventional chromatographic procedures.

4. A process for the preparation of the hPTH fragment-(1-37) or its derivatives according to claim 1, characterized in that the hPTH fragment-(1-37) is prepared by means of the conventional methods of solid-phase and liquid-phase synthesis from the protected amino acids contained in the specified sequence, the resulting product is de-blocked and then purified by the established chromatographic procedures.

5. A medicament, containing the human circulating parathyreoid hormone according to claim 1, the hPTH fragment-(1-37), as active ingredient besides conventional auxiliary materials and additives.

6. Use of the hPTH fragment-(1-37) medicament as well as of its natural and pharmacologically compatible derivatives according to claim 1 for the preparation of a medicament for the treatment of diseases of the parathyroid, and especially the hypofunction thereof (hypoparathyreoidism).

7. Use of the hPTH fragment-(1-37) medicament as well as of its natural and pharmacologically compatible derivatives according to claim 1 for the preparation of a medicament for the treatment of degenerative bone diseases, and especially of osteoporosis.

8. Use of the hPTH fragment-(1-37) medicament as well as of its natural and pharmacologically compatible derivatives according to claim 1 for the preparation of a medicament for the treatment of bone fractures during the healing phase.

9. Use of the hPTH fragment-(1-37) medicament as well as of its natural and pharmacologically compatible derivatives according to claim 1 for the preparation of a medicament for the treatment of cardiovascular diseases associated with an increase in blood pressure, and especially of essential hypertonia.

10. Use of the hPTH fragment-(1-37) medicament as well as of its natural and pharmacologically compatible derivatives according to claim 1 for the preparation of a medicament for the treatment of pulmonary diseases associated with an increase in blood pressure in the pulmonary circulatory system, and especially of primary pulmonal hypertonia.

11. Use of the hPTH fragment-(1-37) medicament as well as of its natural and pharmacologically compatible derivatives according to claim 1 for the preparation of a medicament for the treatment of renal diseases associated with disorders of the electrolyte excretion, and especially of acute renal insufficiency as well as of disorders of the phosphate and calcium excretion.

12. Use of the hPTH fragment-(1-37) medicament as well as of its natural and pharmacologically compatible derivatives according to claim 1 for the preparation of a medicament for the treatment of male impotence.

13. Use of the hPTH fragment-(1-37) medicament according to claim 12 in galenically suitable pharmaceutical preparations, especially in the form of injectable solutions, alone or in combinations with other peptides such as cardiodilatin/urodilatin and/or calcitonin/gene related peptide (CGRP) for the phasic treatment in fertilization care and impotence, the peptide hPTH-(1-37) being applied in single doses between 1 and 10 »g preferably into the male member.

14. Use of the hPTH fragment-(1-37) medicament as well as of its natural and pharmacologically compatible derivatives according to claim 1 for the preparation of a medicament for the diagnosis of diseases, and especially according to any of claims 6 to 13, by preparing specific antibodies against the synthetic peptide and measuring the concentration of hPTH-(1-37) in the blood by means of immunoassays.

15. Use of the hPTH fragment-(1-37) medicament as well as of its natural and pharmacologically compatible derivatives according to claim 1 for the preparation of a medicament in various galenic application forms, especially as a lyophilizate.

16. Use according to claim 15, wherein the galenic application form, and especially the lyophilized form taken-up with mannitol, in sterile vials for dissolution in physiological saline and/or infusion solutions for the repeated individual injection and/or permanent infusion contains amounts of from 300 »g to 30 mg of pure hPTH-(1-37) per therapy unit.

## Revendications

1. Fragment de hPTH (1-37) avec la séquence d'amino-acides 
Ser-Val-Ser-Glu-Ile-Gln-Leu-Met-His-Asn-Leu-
Gly-Lys-His-Leu-Asn-Ser-Met-Glu-Arg-Val-Glu-Trp-Leu-Arg-
Lys-Lys-Leu-Gln-Asp-Val-His-Asn-Phe-Val-Ala-Leu,
 ainsi que ses dérivés naturels et pharmacologiquement acceptables, en particulier des dérivés amidés, acétylés, phosphorylés et glycosylés de hPTH (1-37).

2. Procédé de préparation du fragment de hPTH (1-37) ou de ses dérivés selon la revendication 1, caractérisé en ce qu'on prépare celui-ci par l'intermédiaire d'une expression procaryote ou eucaryote et on le purifie par chromatographie.

3. Procédé de préparation du fragment de hPTH (1-37) ou de ses dérivés selon la revendication 1, caractérisé en ce qu'on l'isole du sang humain, d'une manière connue, au moyen d'un procédé usuel de chromatographie.

4. Procédé de préparation du fragment de hPTH (1-37) ou de ses dérivés selon la revendication 1, caractérisé en ce qu'on prépare le fragment de hPTH (1-37) au moyen des procédés usuels de ---- synthèse en phase solide et de----synthèse en phase liquide à partir des amino-acides protégés qui sont contenus dans la séquence indiquée, on le débloque et on le purifie au moyen des procédés usuels de chromatographie.

5. Médicament contenant l'hormone parathyroïdienne circulante humaine selon la revendication 1, c'est-à-dire le fragment de hPTH (1-37), comme principe actif, avec des adjuvants et des additifs usuels.

6. Utilisation du fragment de hPTH (1-37) ainsi que de ses dérivés naturels et pharmacologiquement acceptables selon la revendication 1 pour la préparation d'un medicament pour le traitement des maladies de la glande parathyroïde, en particulier dans le cas d'une hypofonction de celle-ci (hypoparathyroïdie).

7. Utilisation du fragment de hPTH (1-37) ainsi que de ses dérivés naturels et pharmacologiquement acceptables selon la revendication 1 pour la préparation d'un médicament pour le traitement des maladies osseuses dégénératives, en particulier l'ostéoporose.

8. Utilisation du fragment de hPTH (1-37) ainsi que de ses dérivés naturels et pharmacologiquement acceptables selon la revendication 1 pour la préparation d'un médicament pour le traitement des fractures osseuses pendant la phase de guérison.

9. Utilisation du fragment de hPTH (1-37) ainsi que de ses dérivés naturels et pharmacologiquement acceptables selon la revendication 1 pour la préparation d'un médicament pour le traitement des maladies circulatoires qui s'accompagnent d'une augmentation de la pression sanguine, en particulier l'hypertonie essentielle.

10. Utilisation du fragment de hPTH (1-37) ainsi que de ses dérivés naturels et pharmacologiquement acceptables selon la revendication 1 pour la préparation d'un médicament pour le traitement des maladies pulmonaires qui s'accompagnent d'une augmentation de la pression sanguine dans la circulation pulmonaire, en particulier l'hypertonie pulmonaire primaire.

11. Utilisation du fragment de hPTH (1-37) ainsi que de ses dérivés naturels et pharmacologiquement acceptables selon la revendication 1 pour la préparation d'un médicament pour le traitement des maladies rénales qui s'accompagnent de dysfonctionnements de l'élimination des électrolytes, en particulier dans le cas d'une insuffisance rénale aiguë, ainsi que dans le cas de dysfonctionnements de l'élimination du phosphate et du calcium.

12. Utilisation du fragment de hPTH (1-37) ainsi que de ses dérivés naturels et pharmacologiquement acceptables selon la revendication 1 pour la préparation d'un médicament pour le traitement de l'impuissance masculine.

13. Utilisation du fragment de hPTH (1-37) selon la revendication 12 dans des préparations pharmaceutiques galéniques appropriées, en particulier dans des solutions injectables, individuellement ou combiné avec d'autres peptides, comme la cardiodilatine/urodilatine et/ou des peptides apparentés aux gènes de calcitonine (*Calcitonin-Gene-Related-Peptide*) (CGRP), pour le traitement par phases de la fécondité et de l'impuissance,----------le peptide hPTH (1-37) étant administré en doses individuelles comprises entre 1 et 10 microgrammes, de préférence dans le membre viril.

14. Utilisation du fragment de hPTH (1-37) ainsi que de ses dérivés naturels et pharmacologiquement acceptables selon la revendication 1 pour la préparation d'un médicament pour diagnostiquer des maladies, en particulier selon l'une quelconque des revendications 6 à 13, où on prépare des anticorps spécifiques dirigés contre le peptide synthétique et on mesure la concentration du hPTH (1-37) dans le sang, au moyen d'un essai immunologique.

15. Utilisation du fragment de hPTH (1-37) ainsi que de ses dérivés naturels et pharmacologiquement acceptables selon la revendication 1 pour la préparation d'un médicament sous différentes formes galéniques d'administration, en particulier un lyophilisat.

16. Utilisation selon la revendication 15, où la forme galénique d'administration, en particulier la forme lyophilisée reprise dans du mannitol, contenue dans des ampoules stériles pour une dissolution dans du sérum physiologique et/ou des solutions de perfusion, pour des injections individuelles répétées et/ou une perfusion, contient de 300 microgrammes à 30 milligrammes de hPTH (1-37) pur par unité de thérapie.
